# EUROPEAN PATENT SPECIFICATION

(11) **EP 2 200 586 B1**
(45) Date of publication and mention of the grant of the patent: **13.11.2019**
(21) Application number: 08829918.5
(22) Date of filing: 05.09.2008
(51) Int. Cl.: A61K 9/127

(54) **IMPROVED LIPOSOMES AND USES THEREOF**
VERBESSERTE LIPOSOME UND IHRE VERWENDUNG
LIPOSOMES AMÉLIORÉS ET LEURS UTILISATIONS

(30) Priority: 07.09.2007 EP 07115897
(43) Date of publication of application: 30.06.2010
(73) Proprietor: Synvolux IP B.V., 2333 CH Leiden (NL)
(72) Inventor: KAMPS, Johannes Adrianus Antonius Maria, NL-9736 BE Groningen (NL); MOLEMA, Grietje, NL-9718 HD Groningen (NL); RUITERS, Marcel Herman Josef, NL-9766 PJ Eelderwolde (NL); ADRIAN, Joanna Ewa, NL-9712 SR Groningen (NL)
(74) Representative: V.O.
(86) International application number: PCT/NL2008/050587
(87) International publication number: WO 2009/031896

(56) References cited:
- EP-A- 0 513 878
- EP-A- 0 755 924
- WO-A-2006/043809
- ZUHORN^A I S ET AL: "Interference of serum with lipoplex-cell interaction: modulation of intracellular processing" BIOCHIMICA ET BIOPHYSICA ACTA. BIOMEMBRANES, AMSTERDAM, NL, vol. 1560, no. 1-2, 18 February 2002 (2002-02-18), pages 25-36, XP004342702 ISSN: 0005-2736
- REJMAN J ET AL: "Characterization and transfection properties of lipoplexes stabilized with novel exchangeable polyethylene glycol-lipid conjugates" BIOCHIMICA ET BIOPHYSICA ACTA. BIOMEMBRANES, AMSTERDAM, NL, vol. 1660, no. 1-2, 28 January 2004 (2004-01-28), pages 41-52, XP004486900 ISSN: 0005-2736
- IRENE VAN DER WOUDE TE AL: "Novel pyridinium surfactants for efficient, nontoxic in vitro gene delivery" PROCEEDINGS OF THE NATIONAL ACADEMY OF SCIENCES OF USA, NATIONAL ACADEMY OF SCIENCE, WASHINGTON, DC, US, vol. 94, February 1997 (1997-02), pages 1160-1165, XP002094014 ISSN: 0027-8424
- OBERLE V ET AL: "Lipoplex formation under equilibrium conditions reveals a three-step mechanism" BIOPHYSICAL JOURNAL, NEW YORK, US, US, vol. 79, no. 3, 1 September 2000 (2000-09-01), pages 1447-1454, XP002364029 ISSN: 0006-3495

## Description

The invention relates to the field of molecular medicine and pharmacology. More specifically, it relates to liposomes and their use as delivery vehicle for therapeutic compounds. Also provided is a method for preparing liposomes.

The therapeutic benefit of many compounds is limited by low uptake of the compound by the target cells. Generally, for maximum therapeutic benefit, delivery of the compound to the cytoplasmic or nuclear compartment of the cell is desired, where gene transcription, and translation of mRNA into protein take place. Lipophilic compounds, including many small, uncharged compounds, can permeate across the cell membrane and allow relatively efficient uptake by the cell. However, for a variety of larger and/or charged compounds, such as proteins, nucleic acids, and highly water soluble charged organic compounds, passive uptake by permeation across the cell membrane is limited.

Several methods for improving uptake of such compounds into cells have been proposed. For example, a drug can be administered in a modified or prodrug form for transport into cells and then undergo enzymatic conversion to an active form within the cells. However, it will be understood that the preparation of a suitable prodrug form is not possible for each and every therapeutic compound.

Alternatively, the cellular processes of phagocytosis or endocytosis can be used, where drug-containing particles are taken up by the target cells. With most of the currently available drug delivery particles, this approach is however limited to certain cell types, for example, phagocytosis is limited to cells of monocyte lineage and to certain other myeloid cells, such as neutrophils, while endocytosis is limited in a number of other cells, among other cells from mesenchymal lineage, such as vascular endothelial cells, epithelial cells and fibroblasts. Thus, unlike macrophages and other cells that are specialized in scavenging and processing particles from the blood, there are many cells that are not or much less equipped for handling drug-containing particles.

Still another approach to enhancing drug uptake by cells involves the use of fusogenic particles designed to fuse with the surface membrane of a target cell, releasing the particle's contents into the cytoplasmic compartment of the cell. Inactivated and reconstituted virus particles have been proposed for this purpose, particularly in gene therapy where large nucleic acid strands are introduced into cells. Virus-like particles composed of fusion-promoting viral proteins embedded in an artificial lipid bilayer represent another example. However, safety concerns and the expenses associated with growing, isolating, and deactivating viral components limit a broad application of these approaches.

Endothelial cells, covering the vascular wall of all blood vessels, are an example of cells that are hampered with respect to the handling of drug-containing particles, e.g., drug-loaded liposomes. Endothelial cells play a pivotal role in whole body homeostasis and the patho(physio)logy of important diseases, including (chronic) inflammation and cancer. They are also attractive target cells /target tissue for drug delivery as they are fully accessible for any compound transported by the blood. In addition, the heterogeneity of the endothelium with respect to appearance and function allows for tissue and disease-specific drug delivery approaches. Endothelial cells are a promising target for cancer therapy because angiogenesis is vital for the supply of oxygen and nutrients to solid tumours. Moreover, in inflammatory diseases they guide the movement of white blood cells from the blood into the tissue, and as such are indispensable for disease development. The present inventors observed that liposomes targeted to E-selectin or VCAM-1 expressed on TNFα-activated endothelial cells are readily endocytosed, in amounts that are comparable to the endocytosis capacity of macrophages. However, unlike macrophages, endothelial cells do not extensively process or degrade the liposome and hence accumulate the drug-loaded vehicle inside vesicular bodies. The absence of liposome degradation and/or destabilization results in retention of the encapsulated drug in the liposome and, consequently, in inferior pharmacological efficacy.

Therefore, a goal of the present invention is to provide a lipid-based drug delivery vehicle that allows for an improved intracellular drug release in target cells as compared to known delivery vehicles. A specific aim is to provide a lipid-based drug delivery vehicle for an efficient intracellular drug release in target cells that are not specialized in scavenging and/or processing lipid based-particles, such as endothelial cells and epithelial cells, muscle cells, brain cells, nerve cells, skin cells, hair cells, subsets of progenitor cells, and pericytes and all other cells not specialized in processing delivery vehicles.

Using a method which distinguishes between a) liposome uptake by a target cell and b) drug release from a liposome in a target cell, the present inventors surprisingly found that the incorporation of a synthetic pyridinium-derived amphiphile in the bilayer of a "classical" or "traditional" liposome (i.e. an aqueous compartment surrounded by a lipid bilayer) yields a lipid-based delivery vehicle that ensures efficacious intracellular drug delivery in cells that are not specialized in processing lipid-based particles. Only liposomes comprising a bilayer comprising one or more synthetic pyridinium-derived amphiphile(s) in in an amount of 2 to 25 mol% based on the total lipid content were found to be sufficiently stable. Accordingly, the invention provides a liposome comprising at least one lipid bilayer enclosing an aqueous interior compartment, wherein said lipid bilayer comprises at least one synthetic pyridinium-derived amphiphile having a pyridinium-moiety in its polar group, wherein said at least one synthetic pyridinium-derived amphiphile is a Saint-molecule, and wherein the total amount of pyridinium-derived amphiphile is 2 to 25 mol% based on the total lipid content of the liposome.

The term "liposome" is known in the art to refer to an aqueous compartments enclosed by phospholipid bilayer membrane. Phospholipid molecules consist of an elongated nonpolar (hydrophobic) structure with a polar (hydrophilic) structure at one end. When dispersed in water, they spontaneously form bilayer membranes, also called lamellae, which are composed of two monolayer layer sheets of lipid molecules with their nonpolar (hydrophobic) surfaces facing each other and their polar (hydrophilic) surfaces facing the aqueous medium.

An amphiphile is a compound consisting of molecules having a polar water-soluble group attached to a water-insoluble hydrocarbon chain. Pyridinium refers to the cationic form of pyridine. This can either be due to protonation of the ring nitrogen or because of addition of a substituent to the ring nitrogen, typically via alkylation. The lone pair of electrons on the nitrogen atom of pyridine is not delocalized, and thus pyridine can be protonated easily. The expression "pyridinium-derived" as used herein refers to any amphiphile having a pyridinium-moiety in its polar group.

Synthetic pyridinium-derived amphiphiles are known in the art. Of particular interest for use in the present invention is the group of synthetic pyridinium-derived amphiphiles covered by Synvolux' proprietary technology, SAINT (Synthetic, Amphiphilic, INTeractive). See for instance European Patent EP0755924-B1. At least one synthetic pyridinium-derived amphiphile is selected from the group of SAINT compounds with the general formula (I): 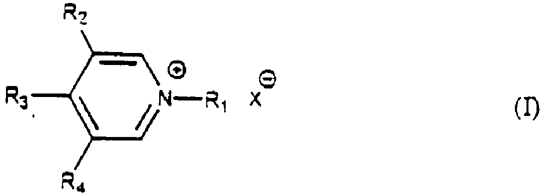 in which:
R₁ is a (C₁-C₅)alkyl, ar(alkyl) or an alkyl group with a cationic functional group, like 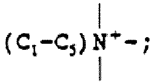
or R₁ is (C₁-C₅ alkylene) R₅ in which R₅ is a structure with the general formula I except R₁;
X is a halide counter ion, chosen from Cl⁻, I⁻, Br⁻;
R₃ is hydrogen and R₂ and R₄ are identical or different and are chosen from the group, comprising branched or linear (C₁₀-C₂₀)alkyl, a mono- or polyunsaturated (C₁₀-C₂₀)alkenyl, O=C-O-alkyl, 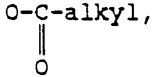
or ar(alkyl),
or R₂ and R₄ are hydrogen and R₃ is -CH(R₅)₂ with R₅, comprising (C₁₀-C₂₀)alkyl, mono- or polyunsaturated (C₁₀-C₂₀)alkenyl, O=C-O-alkyl, 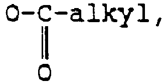
or aralkyl.

In a further embodiment, a SAINT molecule as defined above is incorporated,
wherein disclaimed are the compounds with the general formula I in which R₁ is CH₃, R₂ and R₄ are hydrogen, R₃ is (C₁₆H₃₃)₂CH and X is all mentioned counter ions and disclaimed are the compounds in which R₁ is CH₃, R₂ and R₄ are C₁₆H₃₃-O-C(O), R₃ is hydrogen and X is all mentioned counter ions.

Liposomes according to the present invention comprising a SAINT molecule are also referred to as "SAINT-o-somes". Specific embodiments of the present invention use SAINT-18 (1-methyl-4-(cis-9-dioleyl)methyl-pyridinium-chloride) and/or SAINT-12 (1-methyl-4-(pentacosan-13-yl)-pyridinium-chloride).

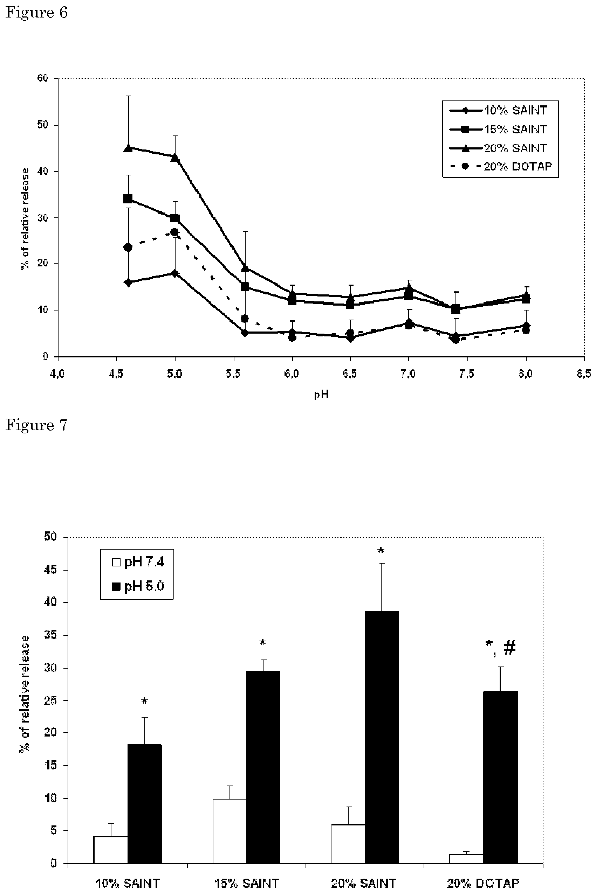

The incorporation of synthetic pyridinium-derived amphiphiles, such as SAINT, in traditional liposomes has not been described or suggested in the art. In contrast, SAINT molecules have thus far been used in non-liposomal delivery vehicles (called "lipoplexes") for the delivery of macromolecules such as nucleic acids or proteins to mammalian cells. EP-A-0755924 for example discloses complexing of the macromolecule to SAINT, optionally in a 1:1 mixture with a helper lipid, to form a lipoplex particle of up to several µm in size. In the lipoplex formed only non-covalent interactions are present between SAINT and the macromolecule. The cationic amphiphiles on the surface of the particle have high affinity for the negatively charged cell surface. Whereas the introductory section of EP-A-0755924 refers briefly to "liposomes which consist of a bilayer of phospholipids", it must be emphasized that the invention disclosed in EP-A-0755924 clearly does not relate to such liposomes. Rather, it relates to the lipoplexes whose structural organization was studied in detail by Oberle et al. using atomic force microscopy (2000, Biophysical Journal 79(3), 1447-1454). Figure 7 of Oberle *et al*. illustrates that the SAINT-containing lipoplexes, as disclosed for example in EP-A-0755924, consist of DNA wrapped into several layers of amphiphile, which subsequently merge into a large complex. It is of note that some DNA "sticks out" of the lipoplex and is this not shielded from the environment as it would be when incorporated in the interior compartment of a SAINT-containing liposome as disclosed in the present invention. Rejman et al. (Biochimica et Biophysica Acte 1660 (2004) 41-52), Zuhorn et al. (Biochmica et Biophysica Acta 1560 (2002) 25-36), Van de Woude et al. (Proc. Natl. Acad. Sci. USA, vol. 94, pp. 1160-1165, 1997) and WO2006/043809 likewise disclose lipoplexes rather than liposomes comprising an aqueous interior compartment.

In contrast, the present invention relates to liposomes comprising an aqueous interior space surrounded by a lipid bilayer, wherein one or more synthetic pyridinium-derived amphiphiles, comprising at least one Saint-molecule, are incorporated. Without wishing to be bound by any theory, the properties of the cationic pyridinium headgroup of the amphiphile makes the liposomal lipid bilayer less rigid and more prone to lipid mixing (e.g. fusing) with intracellular, endosomal membrane lipids, thereby promoting intracellular release of the encapsulated compound. As is demonstrated herein below, this effect is not observed for just any cationic amphiphile, since liposomes with similar amounts of DOTAP (N-[1-(2,3-Dioleoyloxy)]-N,N,N-trimethylammonium propane) a well known cationic liposomal transfection reagent, exhibited inferior performance.

Liposomes according to the invention have one or more pyridinium-derived amphiphiles, comprising at least one Saint-molecule. The exact composition of the liposomes will depend on the particular circumstances for which they are to be used. Those of ordinary skill in the art will find it a routine matter to determine a suitable lipid composition. General information on liposomes can for example be found Lasic, D.D. "Liposomes: from physics to application", Elsevier Science B.V. Amsterdam, 1993 (ISBN 0444895485).

The relative amount of pyridinium-derived amphiphile in a liposome of the invention can vary, but is typically up to about 5-25 mole% based on the total amount of other lipid constituents of the liposome. This ensures that the liposome is stable outside the target cell (e.g., in the blood stream) while it allows for efficient intracellular release of its encapsulated contents upon mixing or fusion with a target cell membrane. Very good results were obtained when liposomes comprising 5-20 mole%, preferably 10-20 mole%, such as 11, 12, 13, 14, 15, 16, 17, 18 19 or 20 mole%, pyridinium-derived amphiphile relative to the other lipids was used. Specific embodiments include liposomes comprising 10-20 mole% SAINT molecules, such as 10, 11, 12, 14, 15, 17, 19 or 20 mole% SAINT-18 or SAINT-12.

In one embodiment, the liposomes of the present invention consist essentially of a single type of lipid in addition to the at least one pyridinium-derived amphiphile. In a preferred embodiment, the liposomes comprise a mixture of two or more lipids, preferably selected from the group consisting of glycerophospholipids, sphingolipids and cholesterol. Naturally occurring and/or synthetic lipids may be used. In fact, any type of liposome-forming lipid or molecule may be used in combination with at least one pyridinium-derived amphiphile, comprising at least one Saint-molecule, to form a liposome according to the invention. The lipid constituting the liposomes of the present invention includes phosphatidylcholines, phosphatidylethanolamines, phosphatidic acids, gangliosides, glycolipids, phosphatidylglycerols, and cholesterol. The phosphatidylcholines preferably include dimyristoylphosphatidylcholine, dipalmitoylphosphatidylcholine, palmitoyloleoylphosphatidylcholine (POPC) and distearoylphosphatidylcholine. The phosphatidylethanolamines preferably include dimyristoylphosphatidylethanolamine, dipalmitoylphosphatidylethanolamine, and distearoylphosphatidylethanolamine. The phosphatidic acids preferably include dimyristoylphosphatidic acid, dipalmitoylphosphatidic acid, distearoylphosphatidic acid, and dicetylphosphoric acid. The gangliosides preferably include ganglioside GM1, ganglioside GD1a, and ganglioside GT1b. The glycolipids preferably include galactosylceramide, glucosylceramide, lactosylceramide, phosphatide, and globoside. The phosphatidylglycerols preferably include dimyristoylphosphatidylglycerol, dipalmitoylphosphatidylglycerol, and distearoylphosphatidylglycerol.

*In vivo* studies have shown that conventional liposomes are rapidly removed from the circulation by cells of the reticuloendothelial system, i.e., tissue-resident phagocytes present in a number of organs, particularly the spleen and the liver. This interaction is reduced when using sterically stabilized liposomes, which can be prepared by insertion of monosialoganglioside (GM1) or poly(ethylene glycol) (PEG) derivatized lipids within the lipid bilayer of liposomes. Such liposomes coated with inert polymers show a substantial improvement in their blood circulation half life (in human in the order of days as opposed to minutes for conventional liposomes).

Sterically stabilized immunoliposomes with cell specific recognition properties are often prepared by coupling of antibodies to the distal ends of PEG chains. Using the PEG chains as linker between the liposome and antibody leads to an enhanced antibody-antigen recognition and binding since the antibody is not shielded by the steric barrier activity of PEG. Several covalent coupling methods have been developed for attaching (derivatized) antibodies at the PEG terminus. They make use of functionalized PEG-lipids with a chemically reactive endgroup such as hydrazide, N-(3'-(pyridyldithio)proprionate, maleimide, succinyl, p-nitrophenylcarbonyl or cyanuric chloride. Thus, a derivatized (e.g., PEGylated) phospholipid may be used to improve the *in vivo* circulation time of a liposome formulation, and/or to allow for coupling of proteins to the liposomal surface. A liposome preferably comprises a mixture of phosphatidylcholine and cholesterol in relative amounts from 1:1 to 2:1 (mole%), optionally in a mixture with one or more natural or synthetic lipids, for example a PEG-ylated phospholipid.

The liposome itself can be produced through any conventional method including a thin film method, a reverse phase evaporation method, an ethanol injection method, and a dehydration-rehydration method. Accordingly, the invention provides a method for preparing a liposome according to the invention, comprising mixing conventional lipids with a suitable amount of at least one synthetic pyridinium-derived amphiphile, and preparing a liposome according to conventional procedures. Also described are liposomes obtainable by a method of the invention.

For example, a mixture of the above-mentioned lipids, from which the solvents have been removed, can be emulsified by the use of a homogenizer, lyophilized, and melted to obtain multilamellar liposomes. Alternatively, unilamellar liposomes can be produced by the reverse phase evaporation method (Szoka and Papahadjopoulos, 1978, Proc. Natl. Acad. Sci. USA 75:4194-4198). Unilamellar vesicles can also be prepared by sonication or extrusion. Sonication is generally performed with a bath-type sonifier, or a Branson tip sonifier at a controlled temperature as determined by the melting point of the lipid.

Following liposome preparation, the liposomes that have not been sized during formation may be sized by extrusion to achieve a desired size range and relatively narrow distribution of liposome sizes. The particle size of the liposome can be controlled through an ultrasonic radiation method, an extrusion method, a French press method, a homogenization method or any other suitable conventional method.

Extrusion may be carried out by biomembrane extruders, such as the Lipex Biomembrane Extruder (Northern Lipids Inc, Vancouver, British Columbia, Canada). Defined pore size in the extrusion filters may generate unilamellar liposomal vesicles of specific sizes. A size range of about 200-400 nm will allow the liposome suspension to be sterilized by filtration through a conventional filter (e.g., a 0.22 micron filter). The filter sterilization method can be carried out on a high throughput basis. The liposomes can also be formed by extrusion through an asymmetric ceramic filter, such as a Ceraflow Microfilter (commercially available from the Norton Company, Worcester, Mass.).

For therapeutic applications, it is preferred that a liposome according to the invention has a size (i.e. diameter) of up to about 300 nm. In one embodiment, the liposome size ranges from between about 20 to 250 nm, preferably 30-200 nm, such as 50, 60, 75, 100, 120, 135, 150 or 180 nm. A wide variety of liposomes may be used in the invention (including oligo- or multilamellar vesicles), but preferably the liposomes are small unilamellar vesicles (SUVs) having an external diameter of from about 30 to about 300 nanometers (nm), most preferably 50 to 150 nm.

A liposome of the invention may comprise one or more other beneficial constituents, for example means for targeting the liposome to a specific cell. A liposome according to the invention preferably comprises at its surface at least one targeting means capable of recognizing and binding to a surface molecule of a particular target cell or an intracellular compartment of a target cell. Site-specific delivery of drugs to diseased cells can lead to increased therapeutic effects and to a significant reduction of toxicity. Drug targeting by antibody-conjugated liposomes or immunoliposomes represents a technology which has been applied to the targeting of specific sites of drug action such as the brain, lung, cancer cells, HIV-infected cells or cells of the immune system. Liposomal targeting means, or targeting ligands, are known in the art, and include proteins, peptides, such as antibodies or fragments thereof, having specificity for surface antigen of a particular target cell or for a specific intracellular compartment, such as mitochondria. Liposomes provided at their surface with a targeting antibody are referred to in the art as immunoliposomes. Site specific targeting is often mediated by the high affinity binding of monoclonal antibodies to their specific antigens. US patent no. 5,258,499 discloses delivery vehicle formulations comprising active agents encapsulated within liposomal vesicles to which are attached protein hormones (ligands) such as interleukin-2. The ligands are capable of showing affinity for specific cell receptors resulting in delivery of the encapsulated active agent to target cells, enabling delivery of active agents to particular cell populations in the treatment of conditions such as immune system disorders. In a preferred embodiment, said targeting means has specificity for the surface of a target cell that is not specialized in scavenging and processing particles, preferably an endothelial cell.

A targeting moiety on a liposome, e.g., a ligand, is preferably internalized by a target cell. In yet another embodiment, a targeting moiety is a ligand that specifically interacts with a tyrosine kinase receptor such as, for example, EGFR, HER2, HER3, HER4, PD-GFR, VEGFR, bFGFR or IGFR receptors. In still another embodiment, the targeting moiety specifically interacts with a growth factor receptor, an angiogenic factor receptor, a transferrin receptor, a scavenger receptor, a cell adhesion molecule, or a vitamin receptor. Exemplary cell-specific ligands include the RGD-peptide, NGR-peptide, ATWLPPR-peptide, APRPG-peptide, SMSIARL-peptide, TAASGVRSMH-peptide, LTLRWVGLMS-peptide, CDSDSDITWDQLWDLMK-peptide, GPLPLR-peptide, HWGF-peptide, recombinant VEGF, antibodies and monoclonal antibodies, bispecific antibodies and single chain fragments against e.g., E-selectin, VCAM-1, endoglin, MHCII, VEGF:VEGFR complex, αᵥβ₃, moc-31, cd-90, and other cellular target epitopes. Furthermore receptor specific ligand such as transferrin, apolipoprotein E, lactoferrin, modified albumins etc.

Said targeting means on a liposome of the invention preferably is the RGD-peptide motif, anti-E-selectin antibody or anti-VCAM-1 antibody.In one embodiment, a liposome comprises at least one targeting ligand that is bound via a linker-molecule to a SAINT-molecule incorporated in the liposomal lipid bilayer. See for exemplary SAINT-linker-ligand molecules WO2006/043811. Preferably, a liposome according to the invention comprises an unmodified (i.e. not comprising a targeting means) SAINT molecule as well as a SAINT molecule to which at least one cell or organelle-specific ligand is bound via a linker.

In view of sterical hindrance, the relative amount of modified versus unmodified SAINT molecule is preferably less than 5%, more preferably less than 1%, such as approximately 0,1%. For example, liposomes are prepared comprising as bilayer constituents 35.8 mol% POPC, 40 mol% cholesterol, 4 mol% PEG-DSPE, 20 mol% SAINT C18 and 0.2 mol% SAINT C18-linker.

As mentioned above, a liposome of the invention comprises an interior space that can be used to deliver one or more molecules of interest, for instance a biologically active compound, to a target cell. For the purposes of this invention, the term "biologically-active compound" is intended to encompass all naturally-occurring or synthetic compounds capable of eliciting a biological response or having an effect, either beneficial or deleterious, including cytotoxic, on biological systems, particularly tissues, cells and cellular organelles. These compounds are intended to include all varieties of drugs, including antibiotic, antibacterial, antiviral, antimycotic, anti-inflammatory, antiproliferative and antineoplastic drugs, and any inhibitor of intracellular signal transduction developed as therapeutics, such as a MAPK or SAPK inhibitor; hormones, including peptide hormones and steroid hormones; genes, recombinant nucleic acids, oligonucleotides or other nucleic acids encoding all or a portion of a mammalian gene, including custom small interfering RNA (also known as short interfering RNA or siRNA) and specially engineered short RNA molecules which can effectively silence the action of microRNA's in regulating gene expression, such as antagomirs, a viral gene or a gene from a microorganism; antigens; enzymes; nutrients; and most particularly any biologically active compound, that is inefficiently taken up by passive permeation across the cell membrane of a target cell of interest. In one aspect, the liposome comprises in its interior compartment a biologically active compound selected from the group consisting of antibiotic, anti bacterial, anti viral, antimycotic, anti-inflammatory, antiproliferative and antineoplastic drugs; hormones; nucleic acids, including genes, recombinant nucleic acids, oligonucleotides, siRNA, a viral gene or a gene from a microorganism; and antigens. Said biologically active compound may have a neutral or positive net charge, for instance a proteinaceous substance.

Further described are compositions comprising a liposome according to the invention. In general, the liposome composition of the present invention is quite stable during storage, e.g., as measured by the percentage of entrapped entity released outside of the liposomes or still maintained inside of the liposomes after a certain time period from the initial loading of the entity inside the liposomes of the present invention. For example, the liposome composition of the present invention is stable at 4° C for at least 6 months, e.g., less than 10% of entrapped entity is released 6 months after the initial loading of the entity. In one embodiment, the liposome composition of the present invention is stable at 4°C for at least 2 years, e.g., less than 20% of entrapped entity is released 2 years after the initial loading of the entity.

According to another embodiment of the present invention, the liposome composition of the present invention can be provided as a pharmaceutical composition containing the liposome composition of the present invention and a pharmaceutically acceptable carrier. Examples of pharmaceutically acceptable carries are normal saline, isotonic dextrose, isotonic sucrose, Ringer's solution, and Hanks' solution. A buffer substance can be added to provide pH optimal for storage stability. For example, pH between about 6.0 and about 7.5, more preferably pH about 6.5, is optimal for the stability of liposome membrane lipids, and provides for excellent retention of the entrapped entities. Histidine, hydroxyethylpiperazine-ethylsulfonate (HEPES), morpholipo-ethylsulfonate (MES), succinate, tartrate, and citrate, typically at 2-20 mM concentration, are exemplary buffer substances. Other suitable carriers include, e.g., water, buffered aqueous solution, 0.4% NaCl, 0.3% glycine, and the like. Protein, carbohydrate, or polymeric stabilizers and tonicity adjusters can be added, e.g., gelatin, albumin, dextran, or polyvinylpyrrolidone. The tonicity of the composition can be adjusted to the physiological level of 0.25-0.35 mol/kg with glucose or a more inert compound such as lactose, sucrose, mannitol, or dextrin. These compositions may be sterilized by conventional, well known sterilization techniques, e.g., by filtration. The resulting aqueous solutions may be packaged for use or filtered under aseptic conditions and lyophilized, the lyophilized preparation being combined with a sterile aqueous medium prior to administration.

The pharmaceutical liposome compositions can also contain other pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. Additionally, the liposome suspension may include lipid-protective agents which protect lipids against free-radical and lipid-peroxidative damages on storage. Lipophilic free-radical quenchers, such as alpha-tocopherol and water-soluble iron-specific chelators, such as ferrioxamine, are suitable.

The concentration of the liposomes of the present invention in the fluid pharmaceutical formulations can vary widely, i.e., from less than about 0.05% usually or at least about 2-10% to as much as 30 to 50% by weight and will be selected primarily by fluid volumes, viscosities, etc., in accordance with the particular mode of administration selected. For example, the concentration may be increased to lower the fluid load associated with treatment. This may be particularly desirable in patients having atherosclerosis-associated congestive heart failure or severe hypertension. Alternatively, liposome pharmaceutical compositions composed of irritating lipids may be diluted to low concentrations to lessen inflammation at the site of administration.

The amount of liposome pharmaceutical composition administered will depend upon several factors, for example the particular therapeutic entity entrapped inside the liposomes, the disease state being treated, the type of liposomes being used, and / or the judgment of the clinician. Generally, the amount of pharmaceutical liposome composition administered will be sufficient to deliver a therapeutically effective dose of the particular therapeutic entity.

The quantity of pharmaceutical liposome composition necessary to deliver a therapeutically effective dose can be determined by routine *in vitro* and *in vivo* methods, common in the art of drug testing. See, for example, D. B. Budman, A. H. Calvert, E. K. Rowinsky (editors). Handbook of Anticancer Drug Development, LWW, 2003. Therapeutically effective dosages for various therapeutic entities are well known to those of skill in the art; and a therapeutic entity delivered via the pharmaceutical liposome composition of the present invention provides at least the same, or 2-fold, 4-fold, or 10-fold higher activity than the activity obtained by administering the same amount of the therapeutic entity in a conventional liposome formulation not comprising at least one synthetic pyridinium-derived amphiphile. Typically the dosages for the liposome pharmaceutical composition of the present invention range between about 0.005 and about 500 mg of the therapeutic entity per kilogram of body weight, most often, between about 0.1 and about 100 mg therapeutic entity/kg of body weight or targeted organ weight.

A pharmaceutical liposome composition of the present invention may be prepared as a topical or an injectable, either as a liquid solution or suspension. However, solid forms suitable for solution in, or suspension in, liquid vehicles prior to injection can also be prepared. The composition can also be formulated into an enteric-coated tablet or gel capsule according to known methods in the art.

The liposome composition of the present invention can be administered in any way which is medically acceptable which may depend on the condition or disease being treated. Possible administration routes include injections, by parenteral routes such as intramuscular, subcutaneous, intravenous, intra-arterial, intraperitoneal, intra-articular, intra-epidural, intrathecal, or others, as well as oral, nasal, ophthalmic, rectal, vaginal, topical, or pulmonary, e.g., by inhalation. For the delivery of liposomal drugs formulated as described, to tumors of the central nervous system, a slow, sustained intracranial infusion of the liposomes directly into the tumor (a convection-enhanced delivery, or CED) is of particular advantage. See Saito, et al., Cancer Research, vol. 64, p. 2572-2579, 2004; Mamot, et al., J. Neuro-Oncology, vol. 68, p. 1-9, 2004. The compositions may also be directly applied to tissue surfaces. Sustained release, pH dependent release, or other specific chemical or environmental condition mediated release administration is also specifically included herein, e.g., by such means as depot injections, or erodible implants.

It is desirable that the average particle diameter is 400 nm or less in the case of its intravenous administration. This is because a liposome having a particle diameter of exceeding 400 nm is cleared by the liver, spleen and the like reticuloendothelial system and the lungs.

Also provided is a method for facilitating the delivery of a biologically active compound to a target cell that is not specialized in scavenging and processing particles from the blood, comprising the intravenous administration of a liposome according to the invention which comprises said biologically active compound.

A further aspect relates to the use of a liposome according to the invention as delivery vehicle to *in vitro* or *in vivo* introduce a substance of interest in a target cell that is not specialized in scavenging and/or processing lipid-based particles. Preferably, said target cell is selected from the group consisting of endothelial cells, epithelial cells, muscle cells, brain cells, nerve cells, skin cells, hair cells, subsets of progenitor cells, and pericytes. More preferably, the target cell is an endothelial cell. A liposome of the invention and liposome-based delivery method for instance facilitate the (targeted) delivery of a biologically active compound to endothelial cells, for instance TNFα-activated endothelial cells. Thus, a liposome as disclosed herein is advantageously used as drug delivery vehicle.

Also provided is the use of a liposome according to the invention comprising at least one biologically active compound, either in its interior space and/or inserted in the lipid bilayer and/or attached covalently or otherwise to one of the liposome constituents, for the manufacture of a medicament for the treatment of cancer, chronic inflammation, tissue regeneration/repair, diabetes or metabolic disease associated cell dysfunction.

In one embodiment, a liposome provided with an EPCAM targeting means and encapsulating a cytostatic compound is used for the manufacture of a medicament for the treatment of cancer. In another embodiment, a liposome provided with an E-selectin targeting means and comprising a p38MAPK inhibitor is used for the manufacture of a medicament for the treatment of glomerulonephritis or reuma.

### LEGENDS TO THE FIGURES

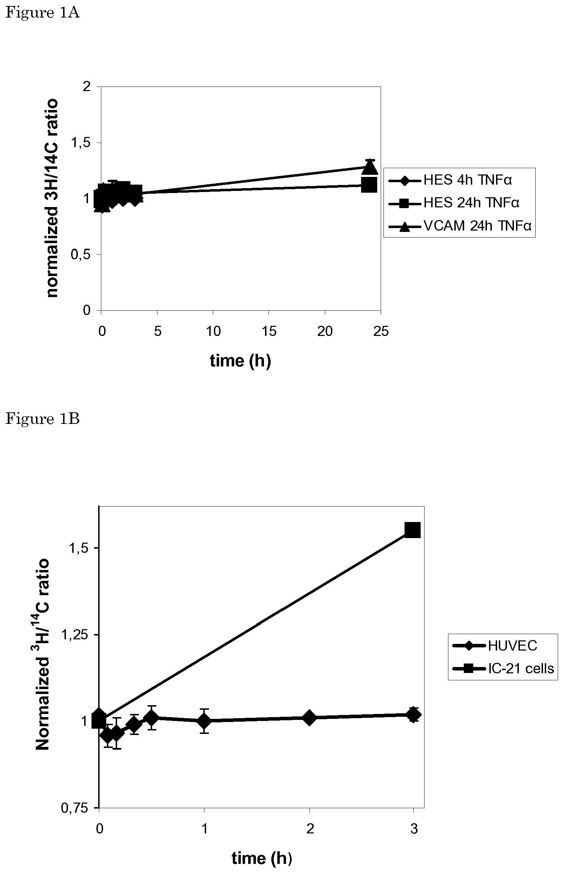

**Figure 1A****:** The intracellular processing of anti-E-selectin immunoliposomes in HUVEC targeted to E-selectin (HES) or VCAM-1 (VCAM) was investigated by using double radiolabeled conventional liposomes, i.e. without synthetic pyridinium-derived amphiphile, containing the metabolically degradable ester cholesteryl [¹⁴C]oleate in addition to the non-degradable [3H]cholesteryloleyl ether. In the case of proper metabolism, following endocytosis the cholesterylester is hydrolyzed and the liberated [¹⁴C]oleic acid will be released from the cells into the culture medium. The [³H]cholesteryloleyl ether will remain associated and thus the ³H/¹⁴C ratio is a convenient measure of liposome degradation [5]. As shown in panel A, the ³H/¹⁴C ratio does not change upon prolonged incubation, indicating the absence of hydrolysis of cholesteryl-[¹⁴C]oleate. Even a 24 h incubation did not result in any significant hydrolysis the double labeled anti-E-selectin immunoliposomes. In panel B, the degradation of immunoliposomes by HUVEC is compared to the degradation of similar liposomes by IC21 cells (peritoneal macrophages). It is shown that the same liposomes can efficiently be degraded by specialized cells. Incubations in the presence of inhibitors (chloroquine, NH₄Cl) of lysosomal degradation indicated that degradation was lysosomal. (data not shown).

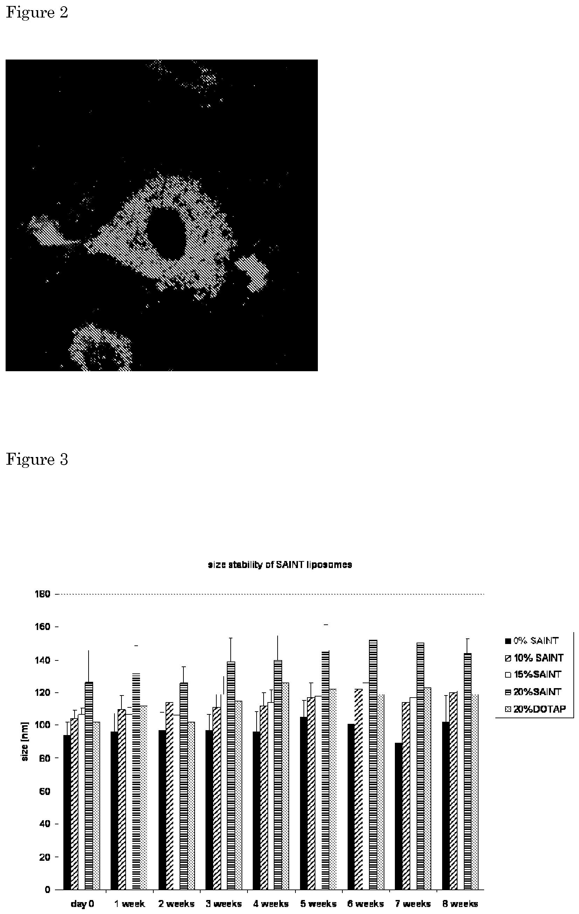

**Figure 2****:** TNFα activated HUVEC were incubated for 24 h with anti-E-selectin immunoliposomes (without synthetic pyridinium-derived amphiphile) that were fluorescently labeled with the lipid bilayer marker DiI. The liposomes have accumulated in distinct intracellular vesicles, while there is no indication of redistribution of the fluorescent label over cellular membranes, i.e., the liposomes are not degraded after being endocytosed by the endothelial cells.
**Figure 3****:** Liposomes according to the invention were prepared as described in the general method and comprising the indicated amount of synthetic pyridinium-derived amphiphile (in this case SAINT-18) or the reference cationic amphiphile DOTAP. Liposomes were stored at 4°C under argon during the time periods indicated. Liposome particle size was analyzed at the indicated times by dynamic light scattering using a Nicomp model 380 submicron particle analyzer in the volume weighing mode. Data are presented as single value for 1, or mean ± sd of 2 to five independent liposome preparations.

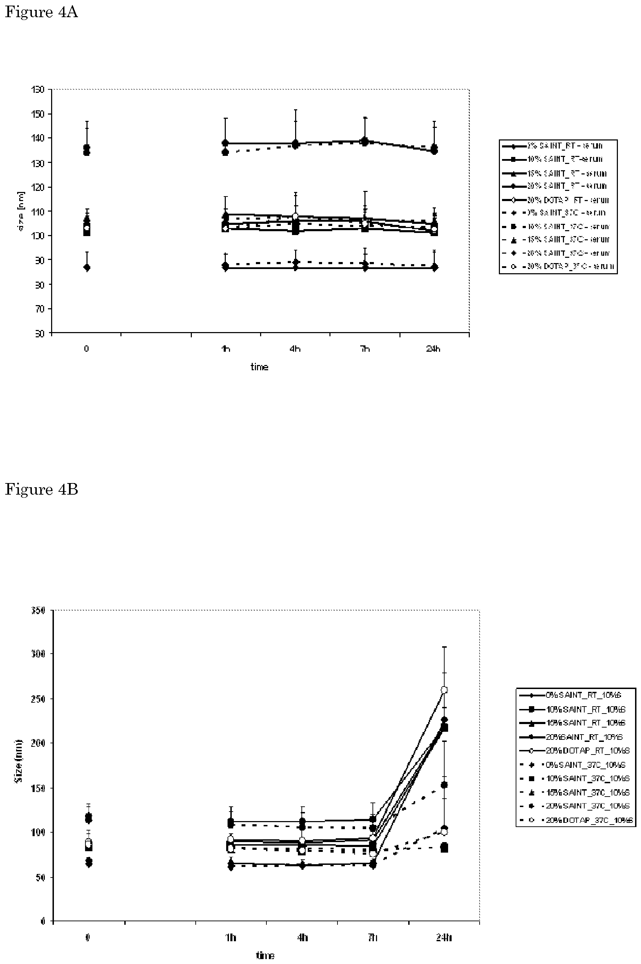

**Figure 4****:** Liposomes were prepared as described in the general method. Liposomes were kept in thermostated water bath at 37°C or on the laboratory bench at room temperature (20°C) in the absence (panel A) or presence (panel B) of 10% (v/v) serum . Particle size was analyzed at the indicated times by dynamic light scattering using a Nicomp model 380 submicron particle analyzer in the volume weighing mode. Data are presented as mean ± sd of 3 or 4 independent liposome preparations.

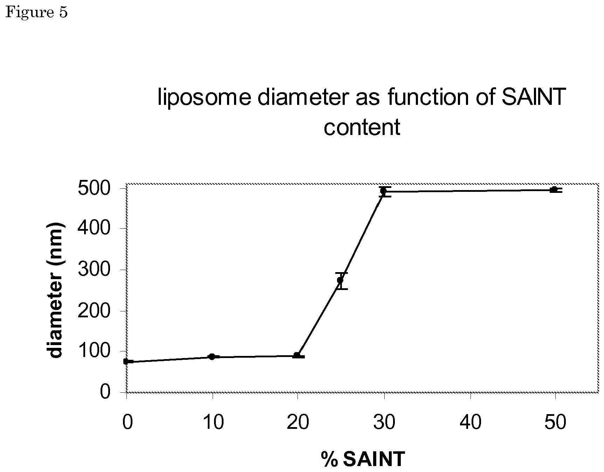

**Figure 5****:** Liposomes were prepared as described with increasing amounts of pyridinium-derived amphiphile. Liposomes are fully size stable when the bilayer is formulated with 0 to 20 mol% SAINT C18. Above around 20 to 25 mol% pyridinium-derived amphiphile, the liposomes start to loose their size stability.
**Figure 6****:** Calcein release from liposomes as a function of pH. Fluorescence of the liposomes was monitored while incubated in buffers with the indicated pH in 96 well plates in a standard fluorimeter. Calcein release was related to total fluorescence of the liposomes measured after treatment with Triton-XlOO. Data are presented as relative release compared to liposomes without SAINT or DOTAP (mean ± sd of 3 to 4 independent experiments).
**Figure 7****:** Calcein release from liposomes formulated with 20 mol% SAINT or DOTAP at pH 7.4 and pH 5. Fluorescence of the liposomes was monitored in buffers with the indicated pH in 96 well plates in a standard fluorimeter. Calcein release was related to total fluorescence of the liposomes measured after treatment with Triton-XlOO. Data are presented as relative release compared to liposomes without SAINT or DOTAP (mean ± sd of 3 to 4 independent experiments). * p <0.005 compared to pH 7.4; # p <0.05 compared to liposomes containing 20 mol% SAINT.

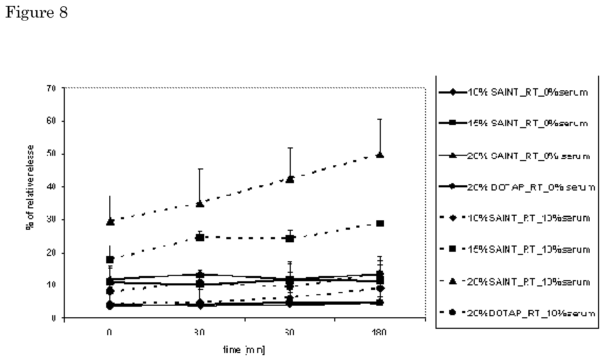

**Figure 8****:** Effect of 10% serum (v/v) on calcein release from liposomes. Fluorescence of the liposomes was monitored in 96 well plates in a standard fluorimeter. Calcein release was calculated after determination of total fluorescence of the liposomes measured after treatment with Triton-XlOO. Data are presented as relative release compared to liposomes without SAINT or DOTAP (mean ± sd of 3 to 4 independent experiments)

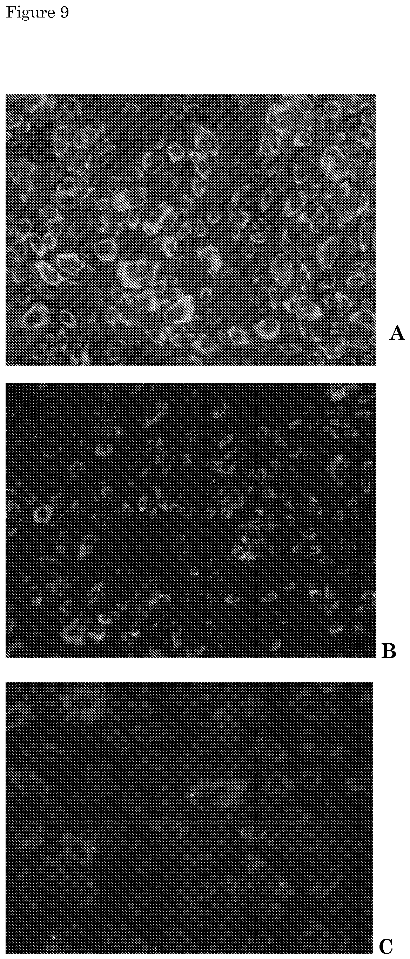

**Figure 9****:** TNFα activated HUVEC were incubated for 24 h with liposomes with encapsulated calcein. Panel A: anti-E-selectin immunoliposomes formulated with 20 mol% SAINT. Panel B: anti-E-selectin immunoliposomes without SAINT. Panel C: untargeted liposomes with 20 mol% SAINT. Calcein release is most prominent with targeted liposomes that contain SAINT.

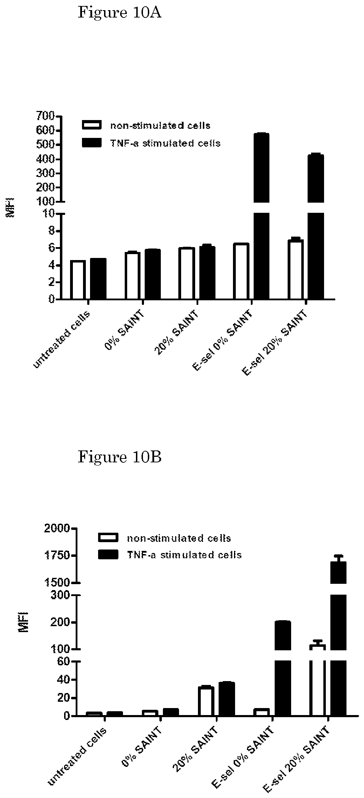

**Figure 10****:** Uptake of DiI (panel A) and calcein (panel B) labeled liposomes by HUVEC. Non- and TNF-α stimulated cells were incubated with the indicated liposomes for 3h. Uptake of liposomes was analyzed by FACS. Data are representative for three independent experiments.

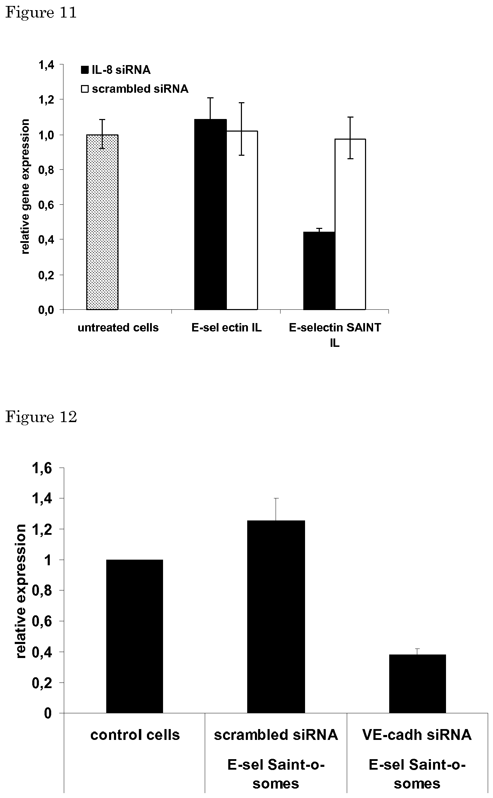

**Figure 11****:** Effect of E-selectin targeted liposomes formulated with 20 mol% SAINT containing Interleukine-8 (IL-8) siRNA on expression of IL-8 in conditionally immortalized human glomerular endothelial cells (ciGEnc). TNF-α activated ciGEnC were incubated with E-selectin SAINT immunoliposomes containing siRNA (either specific for IL-8 or scrambled siRNA) for 48h. Suppression of IL-8 expression was analyzed by real-time PCR. Presented data come from one experiment.
**Figure 12****:** Down-regulation of VE-cadherin expression **Figure 11****:** Effect of E-selectin targeted liposomes formulated with 20 mol% SAINT and containing siRNA on the H5V polyoma middle-T oncogene-transformed endothelioma cell line. E-sel Saint-o-somes containing specific siRNA for VE-cadherin and scrambled siRNA were incubated with H5V cells at the concentration of siRNA 1000 pmol/ml for 48h. The effects of siRNA were analysed by real-time PCR. The expression of VE-cadherin in cells treated with liposomes was compared to TNF-α treated cells (arbitrary set to 1). Data are presented as ±SD.

### EXAMPLES

### Abbreviations used:

TNFα, tumor necrosis factor α
VCAM-1, vascular cell adhesion molecule 1
HUVEC, human umbilical vein endothelial cells
DiI, 1,1'-dioctadecyl-3,3,3',3'-tetramethylindocarbocyanine perchlorate
SAINT, N-methyl-4-alkylpyridium chloride; SATA, (N-succinimidyl-S-acetylthioacetate)
DOTAP, N-[1-(2,3-Dioleoyloxy)]-N,N,N-trimethylammonium propane

### Liposome preparation.

Liposomes were prepared as follows. Lipids from stock solutions of 1-Palmitoyl-2-oleoyl-sn-glycero-3-phosphocholine (POPC), cholesterol (Chol), 2-distearoyl-sn-glycero-3-phosphoethanolamine-N-[methoxy(polyethylene glycol)-2000] (DSPE-PEG) and 1,2-distearoyl-sn-glycero-3-phosphoethanolamine-N-[methoxy(polyethylene glycol)-2000]-maleimide (DSPE-PEG-Mal) in chloroform : methanol (9 : 1, by volume), were mixed in a molar ratio of 55 : 40 : 4 : 1, dried under reduced nitrogen pressure, dissolved in cyclohexane and lyophilized. Where indicated, 1-methyl-4-(cis-9-dioleyl)methyl-pyridinium-chloride (SAINT-18) was added to the lipid mixture in the indicated molar%, always at the cost of the amount of POPC. When appropriate, trace amounts of [3H]cholesteryloleyl-ether and cholesteryl-[¹⁴C]-oleate were added to the preparation as a non-degradable marker and degradable marker, respectively. For fluorescence microscopy and confocal laser scanning microscopy, 0.5 mol% DiI, was added to the lipid mixture as indicated. The lipids were then hydrated in a Hepes buffer (10 mM N-2-hydroxyethylpiperazine-N'-2-ethanesulfonic acid (Hepes), 135 mM NaCl, pH 6.7) or in an aqueous solution containing 100 mM Calcein. The liposomes formed were sized by repeated extrusion (13 times) through polycarbonate filters (Costar, Cambridge MA, USA), pore size 50 nm, using a high pressure extruder (Lipex, Vancouver, Canada).

The monoclonal mouse anti-human E-selectin antibody (H18/7, kindly provided by dr. M. Gimbrone, jr., Boston, MA, USA) was thiolated by means of N-succinimidyl-S-acetylthioacetate and coupled to a maleimide group at the distal end of the polyethylene glycol chain by a sulfhydryl-maleimide coupling technique [2], exactly as described before for albumin [3]. Control immunoliposomes were prepared as described above using irrelevant rat IgG (Sigma-Aldrich Chemie, Zwijndrecht, The Netherlands). Control liposomes without antibody were prepared from the same lipid mixture but, instead of being incubated with antibody, they were incubated with cystein in a molar amount twice that of DSPE-PEG-Mal to block reactive maleimido groups. The immuno-liposomes were characterized by determining protein using mouse immunoglobulin G as a standard 4 and phospholipid phosphorus content 5. Total liposomal lipid concentrations were adjusted for the amount of cholesterol and SAINT present in the liposome preparations.

Particle size was analyzed by dynamic light scattering using a Nicomp model 380 ZLS submicron particle analyzer in the volume weighing mode (NICOMP particle sizing systems, Santa Barbara, CA, USA).

### Cell targeting

HUVEC were activated with TNFα for the indicated time period. Liposomes, either radiolabeled or fluorescently labelled, were added to the cells for the indicated time. For analysis of cell association of radiolabeled liposomes, cells were placed on ice at the end of the incubation, the medium was removed and cells were washed for 5 times with PBS. Cells were then lysed and radioactivity was determined by liquid scintillation counting. For fluorescence microscopy and confocal laser scanning microscopy incubations were performed as described above. Microscopy was performed within 1 hour after finishing the incubation. During this time period there was no change in cell morphology and images of similar incubations were reproducible during this period.

### RESULTS

Immunoliposomes targeted to E-selectin or VCAM-1 on TNFα activated endothelial cells are readily endocytosed in amounts that are comparable to the endocytosis capacity of macrophages. However, unlike macrophages, endothelial cells do not process (degrade) the liposomes (Figure 1) and accumulate them inside vesicular bodies (Figure 2). The absence of liposome degradation and/or destabilization results in retention of the incorporated drugs inside the liposome and consequently in inferior pharmacological efficacy.

Liposomes formulated with increasing mol% SAINT are size stable for a period of over 2 months when stored under argon at 4°C. Formulation of liposomes with SAINT results in a slight increase in particle diameter from 90 nm for liposomes without SAINT to 130 nm for liposomes formulated with 20 mol% SAINT (Figure 3). The results were compared to liposomes containing 20 mol% DOTAP, a non-pyridinium cationic lipid often used in lipid based nucleotide delivery.

Liposomes formulated with SAINT display also size stability at 37°C for at least 24 h (Figure 4A). When incubated in the presence of 10% (v/v) serum, an increase in liposome size after 7 h of incubation, especially at room temperature (Figure 4B). However, at the physiologically relevant temperature of 37°C the increase in diameter after 24 h of incubation was very limited for liposomes formulated with a synthetic pyridinium-derived amphiphile. For liposomes formulated with 20 mol% SAINT the mean diameter increased to 130 nm as compared to time 0.

As can be derived from figure 5, liposomes formulated with up to 20 mol% of pyridinium-derived amphiphile remained size stable. Increasing amounts lead to reduced stability, while at 30 mol% resulted in giant, fused particles having a diameter of around 500 nm.

The release of liposomal content was determined using calcein as a model for a water soluble encapsulated compound. Fluorescence of calcein is quenched at the concentration at which it is encapsulated inside the liposomes (100 mM). Upon dilution calcein emits fluorescence. This allows to distinguish encapsulated calcein (no fluorescence) from released calcein (fluorescence). The endocytotic pathway by which (immuno)liposomes are taken up involves intracellular transport through endosomes, where a drop in pH occurs. Therefore, the release of calcein from liposomes formulated with increasing amounts of synthetic pyridinium-derived amphiphile was determined as a function of pH (see Figure 6). Retention of calcein in liposomes formulated with SAINT is stable at neutral pH. Below pH 6 calcein release increases, where liposomes formulated with 20 mol% show most release in a pH range from 4.5-6. Liposomes formulated with 20 mol% DOTAP display a less favourable release pattern (Figure 6). In Figure 7 the release patterns for liposomes formulated with 20 mol% SAINT are compared at pH 7.4 and pH 5 (relevant pH with regard to pH within endosomes). At pH 5 there is a significant increase in calcein release from the liposomes. Additionally, release from SAINT liposomes at this pH is significantly better than from liposomes formulated with DOTAP.

The addition of 10% serum (v/v) to liposomes formulated with SAINT led to an increased calcein release in time for liposomes containing > 10mol% SAINT. (Figure 8).

Next, *in vitro* calcein release from liposomes was invenstigated. Endothelial cells, HUVEC, were activated with TNFα and incubated with (a) anti-E-selectin immunoliposomes formulated with 20 mol% SAINT, (b) control anti-E-selectin immunoliposomes without SAINT or (c) liposomes (without anti-E-selectin) formulated with 20 mol% SAINT. Figure 9 clearly shows that cells incubated with immunoliposomes formulated with SAINT showed the most calcein fluorescence, indicating preferential intracellular release of the fluorophore from the SAINT liposomes that were taken up by the cells. Uptake of anti-E-selectin immunoliposomes is not influenced by incorporation of SAINT (determined by FACS using DiI as a label, see figure 10), thus the amount of liposomes taken up in Figure 9A and Figure 9B was similar. Liposomes formulated with SAINT but without cell targeting means are taken up to a low extent (Figure 9C).

The result from figure 9 was confirmed in short term incubations with liposomes that were double labeled with DiI as a lipid label and calcein as encapsulated label. HUVEC that were activated for 4 h with TNFα were incubated for 5, 15, 30 or 60 minutes with anti-E-selectin immunoliposomes without SAINT or formulated with 20 mol% SAINT. In cells incubated with liposomes without SAINT primarily red fluorescence is observed at all time points. In contrast, in cells incubated with liposomes formulated with SAINT, apart from red fluorescence also yellow fluorescence was observed at the early time points. The yellow fluorescence indicates release of calcein which fluorescence is co-localized with the liposomes. At 30 and 60 minutes there was also green fluorescence visible, indicating a different intracellular routing of the liposome content as compared to the liposome itself (data not shown).

Uptake of liposomes and release of encapsulated calcein from liposomes was (semi)quantified using FACS. Uptake of E-selectin targeted liposmes by TNFα activated HUVEC was comparable for liposomes that were formulated with 20 mol% SAINT and for liposomes without SAINT, as determined by measurement of the mean fluorescence intensity (arbitrary units) of liposomal DiI (figure 10A). Calcein release from E-selectin targeted liposomes formulated with 20 mol% SAINT was, however, 8-10 times higher as compared to liposomes without SAINT (figure 10B).

E-selectin targeted liposomes formulated with 20 mol% SAINT, containing specific siRNA were very effective in downregulation of target genes (figures 11 and 12). In TNFα activated glomerular endothelial cells E-selectin targeted liposomes formulated with 20 mol% SAINT and containing siRNA against interleukin-8 (IL-8), inhibited IL-8 expression for almost 60%, while there was no effect of liposomes formulated without SAINT or liposomes containing a control (scrambled) siRNA (figure 11). The same holds true for the expression of VE-cadherin, which could be efficiently inhibited for more than 60% using E-selectin targeted liposomes formulated with 20 mol% SAINT, in a mouse derived endothelial cell line.

### References

(1) Bevilacqua MP, Pober JS, Mendrick DL, Cotran RS, Gimbrone MA, Jr. Identification of an inducible endothelial-leukocyte adhesion molecule. Proc Natl Acad Sci USA. 1987;84:9238-9242.
(2) Derksen J, Scherphof G. An improved method for the covalent coupling of proteins to liposomes. Biochimica et Biophysica Acta (BBA) - Biomembranes. 1985;814:151-155.
(3) Kamps JA, Swart PJ, Morselt HW et al. Preparation and characterization of conjugates of (modified) human serum albumin and liposomes: drug carriers with an intrinsic anti-HIV activity. Biochim Biophys Acta. 1996;1278:183-190.
(4) Peterson GL. A simplification of the protein assay method of Lowry et al. which is more generally applicable. Anal Biochem. 1977;83:346-356.
(5) Böttcher CJF, Van Gent CM, Pries C. A rapid and sensitive sub-micro phosphorus determination. Anal Chim Acta. 1961;24:203-204.

## Claims

1. A liposome comprising at least one lipid bilayer enclosing an interior aqueous compartment, wherein said lipid bilayer comprises at least one synthetic pyridinium-derived amphiphile having a pyridinium-moiety in its polar group, wherein said at least one synthetic pyridinium-derived amphiphile is a Saint-molecule, and wherein the total amount of pyridinium-derived amphiphile is 2 to 25 mol% based on the total lipid content of the liposome.

2. Liposome according to claim 1, wherein the total amount of pyridinium-derived amphiphile is 5 to 20 mol%.

3. Liposome according to claim 2, wherein the total amount of pyridinium-derived amphiphile is 10-20 mole%.

4. Liposome according to any one of the preceding claims, having an external diameter of from about 30 to about 300 nanometers (nm).

5. Liposome according to claim 4, having an external diameter of from 50 to 150 nm.

6. Liposome according to any one of the preceding claims, comprising Saint-18 (1-methyl-4-(cis-9-dioleyl)methyl-pyridinium-chloride) and/or Saint-12 (1-methyl-4-(pentacosan-13-yl)-pyridinium-chloride).

7. Liposome according to any one of claims 1 to 6, being sterically stabilized, preferably by insertion of a monosialoganglioside (GM1) and/or or poly(ethylene glycol) (PEG) derivatized lipid within the lipid bilayer.

8. Liposome according to any one of the preceding claims, comprising at its surface at least one targeting means capable of recognizing and binding to a surface molecule of a particular target cell or an intracellular compartment of a target cell.

9. Liposome according to claim 8, wherein said targeting means has specificity for the surface of a target cell that is not specialized in scavenging and processing particles, preferably an endothelial cell.

10. Liposome according to any one of claims 7 to 9, wherein said targeting means is the RGD-peptide motif, anti-E-selectin antibody or anti-VCAM-1 antibody.

11. Liposome according to any one of claims 7 to 10, wherein said at least one targeting means is bound via a linker molecule to a Saint-molecule inserted in the lipid bilayer.

12. Liposome according to any one of the preceding claims, comprising in its interior compartment and/or associated with its lipid bilayer at least one biologically active compound.

13. Liposome according to claim 12, wherein said biologically active compound is selected from the group consisting of antibiotic, antibacterial, antiviral, antimycotic, anti-inflammatory, antiproliferative and antineoplastic drugs; hormones; nucleic acids, including genes, recombinant nucleic acids, oligonucleotides, siRNA, a viral gene or a gene from a microorganism; and antigens.

14. A method for preparing a liposome according to any one of claims 1-13, comprising mixing conventional lipids with a suitable amount of at least one synthetic pyridinium-derived amphiphile, and preparing a liposome according to conventional procedures.

15. A pharmaceutical composition comprising a liposome according to claim 12 or 13 and a pharmaceutically acceptable carrier.

16. Use of a liposome according to any one of claims 1-13 as delivery vehicle to *in vitro* or *in vivo* introduce a substance of interest in a target cell that is not specialized in scavenging and/or processing lipid-based particles, in particular wherein said target cell is selected from the group consisting of endothelial cells, epithelial cells, muscle cells, brain cells, nerve cells, skin cells, hair cells, subsets of progenitor cells, and pericytes.

17. Use of a liposome according to any one of claims 1-13 as a drug delivery vehicle.

18. Use of a liposome according to claims 12 or 13 for the manufacture of a medicament for the treatment of cancer, chronic inflammation, atherosclerosis, tissue regeneration/repair, diabetes or metabolic disease associated cell dysfunction.

19. Use according to claim 18, the liposome being provided at its surface with an EPCAM (Epithelial cell adhesion molecule) targeting means and encapsulating a cytostatic compound for the manufacture of a medicament for the treatment of cancer.

20. Use according to claim 18, the liposome being provided at its surface with an E-selectin targeting means and comprising a p38MAPK inhibitor for the manufacture of a medicament for the treatment of glomerunephritis or reuma.

## Patentansprüche

1. Liposom, umfassend wenigstens eine Lipiddoppelschicht, umschließend ein inneres wässriges Kompartiment, wobei die Lipiddoppelschicht wenigstens ein synthetisches Pyridinium-abgeleitetes Amphiphil mit einer Pyridinium-Einheit in seiner polaren Gruppe umfasst, wobei das wenigstens eine synthetische Pyridinium-abgeleitete Amphiphil ein Saint-Molekül ist, und wobei die Gesamtmenge des Pyridinium-abgeleiteten Amphiphils 2 bis 25 Mol-%, bezogen auf den Gesamtlipidgehalt des Liposoms, ist.

2. Liposom nach Anspruch 1, wobei die Gesamtmenge des Pyridinium-abgeleiteten Amphiphils 5 bis 20 Mol-% ist.

3. Liposom nach Anspruch 2, wobei die Gesamtmenge des Pyridinium-abgeleiteten Amphiphils 10 - 20 Mol-% ist.

4. Liposom nach einem der vorhergehenden Ansprüche, mit einem Außendurchmesser von von etwa 30 bis etwa 300 Nanometer (nm).

5. Liposom nach Anspruch 4, mit einem Außendurchmesser von von 50 bis 150 nm.

6. Liposom nach einem der vorhergehenden Ansprüche, umfassend Saint-18 (1-Methyl-4-(cis-9-dioleyl)methyl-pyridiniumchlorid) und/oder Saint-12 (1-Methyl-4-(pentacosan-13-yl)pyridiniumchlorid).

7. Liposom nach einem der Ansprüche 1 bis 6, das sterisch stabilisiert ist, vorzugsweise durch Insertion eines Monosialogangliosid (GM1) und/oder Poly(ethylenglycol)(PEG) derivatisierten Lipids innerhalb der Lipiddoppelschicht.

8. Liposom nach einem der vorhergehenden Ansprüche, umfassend an seiner Oberfläche wenigstens ein Targeting-Mittel, geeignet zum Erkennen und Binden an ein Oberflächenmolekül einer bestimmten Zielzelle oder ein intrazelluläres Kompartiment einer Zielzelle.

9. Liposom nach Anspruch 8, wobei das Targeting-Mittel eine Spezifität für die Oberfläche einer Zielzellehat, die nicht auf das Auffangen und Verarbeiten von Partikeln vorzugsweise einer Endothelzelle, spezialisiert ist,.

10. Liposom nach einem der Ansprüche 7 bis 9, wobei das Targeting-Mittel das RGD-Peptidmotiv, der Anti-E-Selectin-Antikörper oder Anti-VCAM-1-Antikörper ist.

11. Liposom nach einem der Ansprüche 7 bis 10, wobei das wenigstens eine Targeting-Mittel über ein Linkermolekül an ein Saint-Molekül, eingefügt in die Lipiddoppelschicht, gebunden ist.

12. Liposom nach einem der vorhergehenden Ansprüche, umfassend in seinem inneren Kompartiment und/oder assoziiert mit seiner Lipiddoppelschicht wenigstens eine biologisch aktive Verbindung.

13. Liposom nach Anspruch 12, wobei die biologisch aktive Verbindung ausgewählt ist aus der Gruppe, bestehend aus Antibiotikum, antibakteriellen, antiviralen, antimykotischen, entzündungshemmenden, antiproliferativen und antineoplastischen Arzneimitteln; Hormonen; Nukleinsäuren, einschließlich Genen, rekombinanten Nukleinsäuren, Oligonukleotiden, siRNA, einem viralen Gen oder einem Gen von einem Mikroorganismus; und Antigenen.

14. Verfahren zum Herstellen eines Liposoms nach einem der Ansprüche 1 - 13, umfassend Mischen konventioneller Lipide mit einer geeigneten Menge wenigstens eines synthetischen Pyridinium-abgeleiteten Amphiphils, und Herstellen eines Liposoms nach konventionellen Verfahren.

15. Pharmazeutische Zusammensetzung, umfassend ein Liposom nach Anspruch 12 oder 13 und einen pharmazeutisch akzeptablen Träger.

16. Verwendung eines Liposoms nach einem der Ansprüche 1 - 13 als Abgabevehikel, um *in vitro* oder *in vivo* eine Substanz von Interesse in eine Zielzelle, die nicht auf das Auffangen und/oder Verarbeiten von lipid-basierten Partikeln spezialisiert ist, einzubringen, insbesondere wobei die Zielzelle ausgewählt ist aus der Gruppe, bestehend aus Endothelzellen, Epithelzellen, Muskelzellen, Gehirnzellen, Nervenzellen, Hautzellen, Haarzellen, Untergruppen von Vorläuferzellen, und Perizyten.

17. Verwendung eines Liposoms nach einem der Ansprüche 1 - 13 als ein Arzneimittel-Abgabevehikel.

18. Verwendung eines Liposoms nach den Ansprüchen 12 oder 13 für die Erzeugung eines Medikaments für die Behandlung von Krebs, chronischer Entzündung, Atherosklerose, Geweberegeneration/-reparatur, Diabetes oder Stoffwechselerkrankungen assoziierter Zellfunktionsstörung.

19. Verwendung nach Anspruch 18, wobei das Liposom an seiner Oberfläche mit einem EPCAM (Epithelzellen-Adhäsionsmolekül) Targeting-Mittel bereitgestellt ist, und einkapseln einer zytostatischen Verbindung für die Erzeugung eines Medikaments für die Behandlung von Krebs.

20. Verwendung nach Anspruch 18, wobei das Liposom an seiner Oberfläche mit einem E-Selectin-Targeting-Mittel bereitgestellt ist, und umfassend einen p38MAPKInhibitor für die Erzeugung eines Medikaments für die Behandlung von Glomerunephritis oder Rheuma.

## Revendications

1. Liposome comprenant au moins une bicouche lipidique renfermant un compartiment aqueux intérieur, dans lequel ladite bicouche lipidique comprend au moins un amphiphile dérivé de pyridinium synthétique ayant un fragment pyridinium dans son groupe polaire, dans lequel ledit au moins un amphiphile dérivé de pyridinium synthétique est une molécule Saint, et dans lequel la quantité totale de l'amphiphile dérivé de pyridinium est de 2 à 25 % en moles par rapport à la teneur totale en lipides du liposome.

2. Liposome selon la revendication 1, dans lequel la quantité totale d'amphiphile dérivé de pyridinium est de 5 à 20 % en moles.

3. Liposome selon la revendication 2, dans lequel la quantité totale d'amphiphile dérivé de pyridinium est de 10 à 20 % en moles.

4. Liposome selon l'une quelconque des revendications précédentes, ayant un diamètre externe d'environ 30 à environ 300 nanomètres (nm).

5. Liposome selon la revendication 4, ayant un diamètre externe de 50 à 150 nm.

6. Liposome selon l'une quelconque des revendications précédentes, comprenant Saint-18 (chlorure de 1-méthyl-4-(cis-9-dioléyl)méthylpyridinium) et/ou Saint-12 (chlorure de 1-méthyl-4-(pentacosan-13-yl)pyridinium).

7. Liposome selon l'une quelconque des revendications 1 à 6, qui est stabilisée stériquement, de préférence par insertion d'un monosialoganglioside (GM1) et/ou d'un lipide dérivé au polyéthylèneglycol (PEG) dans la bicouche lipidique.

8. Liposome selon l'une quelconque des revendications précédentes, comprenant à sa surface au moins un moyen de ciblage capable de reconnaître et de se lier à une molécule de surface d'une cellule cible particulière ou un compartiment intracellulaire d'une cellule cible.

9. Liposome selon la revendication 8, dans lequel ledit moyen de ciblage a une spécificité pour la surface d'une cellule cible qui n'est pas spécialisée dans le piégeage et la transformation de particules, de préférence d'une cellule endothéliale.

10. Liposome selon l'une quelconque des revendications 7 à 9, dans lequel ledit moyen de ciblage est le motif peptidique RGD, un anticorps anti-sélectine E ou un anticorps anti-VCAM-1.

11. Liposome selon l'une quelconque des revendications 7 à 10, dans lequel ledit au moins un moyen de ciblage est lié via une molécule de lieur à une molécule Saint insérée dans la bicouche lipidique.

12. Liposome selon l'une quelconque des revendications précédentes, comprenant, dans son compartiment intérieur et/ou en association avec sa bicouche lipidique, au moins un composé biologiquement actif.

13. Liposome selon la revendication 12, dans lequel ledit composé biologiquement actif est choisi dans l'ensemble constitué par les médicaments antibiotiques, antibactériens, antiviraux, antimycotiques, anti-inflammatoires, antiprolifératifs et antinéoplasiques ; les hormones ; les acides nucléiques, y compris les gènes, les acides nucléiques recombinés, les oligonucléotides, les ARNsi, un gène viral ou un gène provenant d'un microorganisme ; et les antigènes.

14. Procédé pour préparer un liposome selon l'une quelconque des revendications 1 à 13, comprenant le mélange de lipides conventionnels avec une quantité convenable d'au moins un amphiphile dérivé de pyridinium synthétique, et la préparation d'un liposome conformément à des procédures conventionnelles.

15. Composition pharmaceutique comprenant un liposome selon la revendication 12 ou 13 et un véhicule pharmaceutiquement acceptable.

16. Utilisation d'un liposome selon l'une quelconque des revendications 1 à 13 en tant que véhicule de délivrance pour introduire in vitro ou in vivo une substance présentant un intérêt dans une cellule cible qui n'est pas spécialisée dans le piégeage et/ou la transformation de particules à base de lipides, en particulier dans laquelle ladite cellule cible est choisie dans l'ensemble constitué par les cellules endothéliales, les cellules épithéliales, les cellules musculaires, les cellules cérébrales, les cellules nerveuses, les cellules cutanées, les cellules capillaires, les sous-ensembles de cellules progénitrices, et les péricytes.

17. Utilisation d'un liposome selon l'une quelconque des revendications 1 à 13 en tant que véhicule de délivrance de médicament.

18. Utilisation d'un liposome selon la revendication 12 ou 13 pour la fabrication d'un médicament destiné au traitement d'un cancer, d'une inflammation chronique, d'une athérosclérose, d'une régénération/ réparation tissulaire, d'un diabète ou d'un dysfonctionnement cellulaire associé à une maladie métabolique.

19. Utilisation selon la revendication 18, dans laquelle le liposome est doté sur sa surface d'un moyen de ciblage EPCAM (molécule d'adhésion cellulaire épithéliale) et encapsule un composé cytostatique, pour la fabrication d'un médicament destiné au traitement d'un cancer.

20. Utilisation selon la revendication 18, dans laquelle le liposome est doté sur sa surface d'un moyen de ciblage de sélectine E et comprend un inhibiteur de p38MAPK, pour la fabrication d'un médicament destiné au traitement d'une gloméruronéphrite ou un rhumatisme.
